# EUROPEAN PATENT APPLICATION

(11) **EP 4 704 112 A1**
(43) Date of publication of application: **04.03.2026**
(21) Application number: 24382933.0
(22) Date of filing: 30.08.2024
(51) Int. Cl.: G16H 50/20, G16H 50/70

(54) **PATIENT SIMILARITY**

(71) Applicant: FUJITSU LIMITED, Kawasaki-shi, Kanagawa 211-8588 (JP)
(72) Inventor: MARTINEZ SANCHEZ, Ander, 28039 Madrid (ES)
(74) Representative: Haseltine Lake Kempner LLP

(57) **Abstract**

A computer-implemented method comprising: using an ML model to predict a prognosis for a target patient based on medical history information relating to the target patient, the medical history information relating to the target patient including an indication of a treatment to be given to the target patient, wherein the ML model has been trained on training data comprising medical history information relating to a plurality of subjects; comparing modified medical history information relating to the target patient, comprising the medical history information relating to the target patient and the predicted prognosis, with a plurality of medical history instances; based on the comparison, selecting at least one medical history instance most similar to the modified medical history information relating to the target patient; and outputting information indicating the predicted prognosis and the selected at least one medical history instance.

## Description

The present invention relates to patient similarity, and in particular to a computer-implemented method, a computer program, an information programming apparatus, and a system.

Early and accurate diagnosis of a disease or condition is an important facet of modern medicine. To help a patient after early disease prediction it is useful to know how to treat the condition or disease that has been diagnosed. It is sometimes difficult to select a treatment for a condition or disease from among the many possible treatments that could be carried out. With the ever-increasing amount of medical data available, a way to use this data in selecting a treatment is desirable.

In light of the above, a method for predicting a prognosis is desired.

The present invention is defined by the independent claims, to which reference should now be made. Specific embodiments are defined in the dependent claims.

According to an embodiment of a first aspect there is disclosed herein a computer-implemented method comprising using a machine learning, ML, model to predict a prognosis for a target patient based on medical history information relating to the target patient, the medical history information relating to the target patient including an indication of a (proposed) treatment to be given to the target patient, wherein the ML model has been trained on training data comprising medical history information relating to a plurality of subjects; comparing modified medical history information relating to the target patient, comprising the medical history information relating to the target patient and the predicted prognosis, with a plurality of medical history instances, each medical history instance comprising medical history information from among the training data and relating to a subject among the plurality of subjects (and to a time period); based on the comparison, selecting at least one medical history instance most similar to the modified medical history information relating to the target patient; and outputting information indicating the predicted prognosis and the selected at least one medical history instance.

Reference will now be made, by way of example, to the accompanying drawings, in which:
Figure 1 is a diagram illustrating a methodology overview;
Figure 2 is a diagram illustrating a machine learning model;
Figure 3 is a diagram illustrating an encoder model;
Figure 4 is a diagram illustrating a method;
Figure 5 is a diagram illustrating a method; and
Figure 6 is a diagram illustrating a computing device.

The following terms may be used herein (this list of definitions is not exhaustive):
- EHR: Electronic Health Record.
- Preventive Medicine: is a medical specialty which focuses on the health of individuals and communities. The goal of preventive medicine is to promote health and well-being and prevent disease, disability and death.
- Diagnosis prediction and Prognosis prediction: While the goal of diagnosis prediction is to calculate the probability that a patient currently has a disease, the prognosis prediction calculates the probability that a condition occurs in the future, for example as a result of a treatment.
- Clinical Decision Support System (CDSS): Are used to support clinicians in their decision making process. Clinical Decision Support (CDS) can be of various natures. In our case, we focus on Prognosis Prediction CDS.
- Black box system: A "black box" is a system whose internal workings are not directly observable or understandable from the outside. In other words, the inputs and outputs of the system are known, but the inner mechanisms that transform the inputs into outputs are not transparent or easily interpretable. Deep Learning models are sometimes considered to be "black boxes".
- Machine Learning (ML): the subfield of computer science that "gives computers the ability to learn without being explicitly programmed". It explores the study and construction of algorithms that can learn from and make predictions on data.
- Artificial Neural Network/Neural Network (ANN/NN): a branch of ML inspired by the way biological nervous systems, such as the brain, process information. They may be used to extract patterns and detect trends that are too complex to be noticed by either humans or other computer techniques.
- Deep Learning (DL): A subset of ML using ANNs to model the data, "deep" meaning multiple layers of neural networks are stacked. This "deepness" contributes to achieving better results in a variety of problems but also makes it more difficult to reason about why a particular prediction was produced.

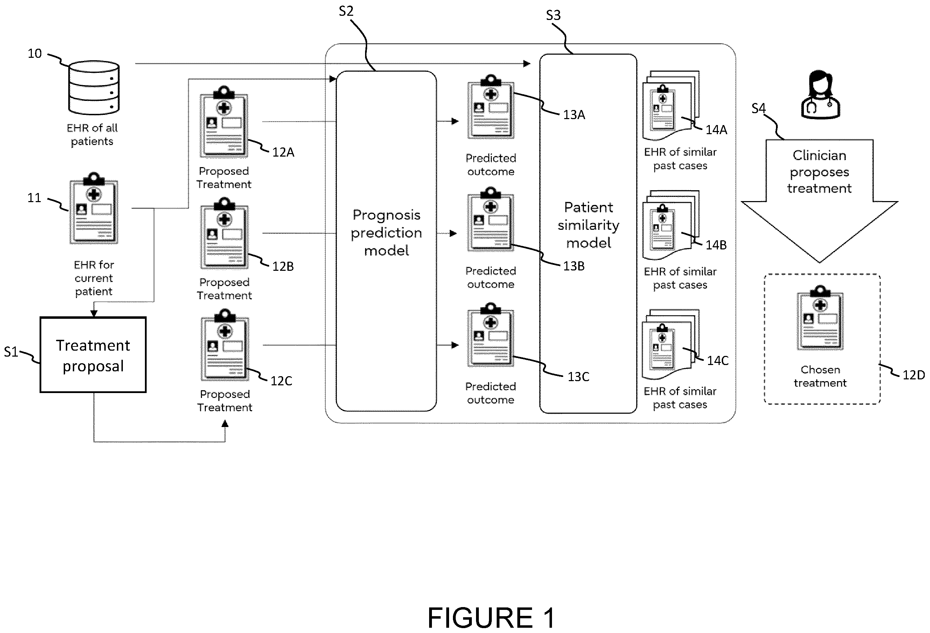

Figure 1 is a diagram illustrating an overview of a methodology disclosed herein. At step S1, at least one treatment is proposed. In Figure 1 three treatments are proposed, treatments 12A, 12B, and 12C. The treatments are proposed based on the EHR 11 of a "current patient", which may be referred to as a target patient. The treatment proposal may be implemented by a clinician/doctor and/or by a model designed/trained to predict treatments.

At step S2, a prognosis prediction model (also referred to herein as an ML model) is used to predict a prognosis for the at least one proposed treatment. In Figure 1, three outcomes 13A, 13B, and 13C are predicted corresponding to the three proposed treatments 12A, 12B, and 12C. The prognosis prediction model predicts the at least one prognosis based on the EHR 11 of the target patient and based on the at least one proposed treatment. The prognosis prediction model is (prior to its use) trained on EHR data e.g. in an unsupervised manner. As an example, the prognosis prediction model could predict that a male patient (with all his background data recorded in his EHR) would develop symptom S1 after one week and disease D1 after one month if went through treatment T1.

At step S3, a patient similarity model is used to find at least one medical history instance most similar to the at least one predicted prognosis. For example, as shown in Figure 1, a plurality of similar EHRs 14A, 14B, and 14C are selected corresponding to each predicted prognosis 13A, 13B, 13C. The patient similarity model compares the target patient's medical history information and at least one predicted prognosis with EHRs 10 of a plurality of other patients/subjects. As described in more detail below, rather than EHRs being selected, at least one medical history instance may be selected, a medical history instance comprising a number of events relating to a subject.

The EHRs 10 of the plurality of subjects may be considered a plurality of medical history instances. The EHRs 10 of the plurality of subjects may be used as training data for the prognosis prediction model and/or the patient similarity model.

At step S4 a clinician proposed a chosen treatment 12D, e.g. chosen from the proposed treatments 12A, 12B, 12C based on the predicted prognoses 13A-C and based on the similar medical history instances 14A-C identified. The identification of similar medical history instances supports the clinician in choosing a treatment by improving their trust in the predicted prognoses and because the clinician may study the similar medical history instances in more detail to help them choose a treatment.

As mentioned above, Figure 1 illustrates a methodology overview. Relevant parts of the overview are described in more detail below. Disclosed herein are methods including steps/operations corresponding to steps S2 and S3 and not necessarily including step S1 or step S4.

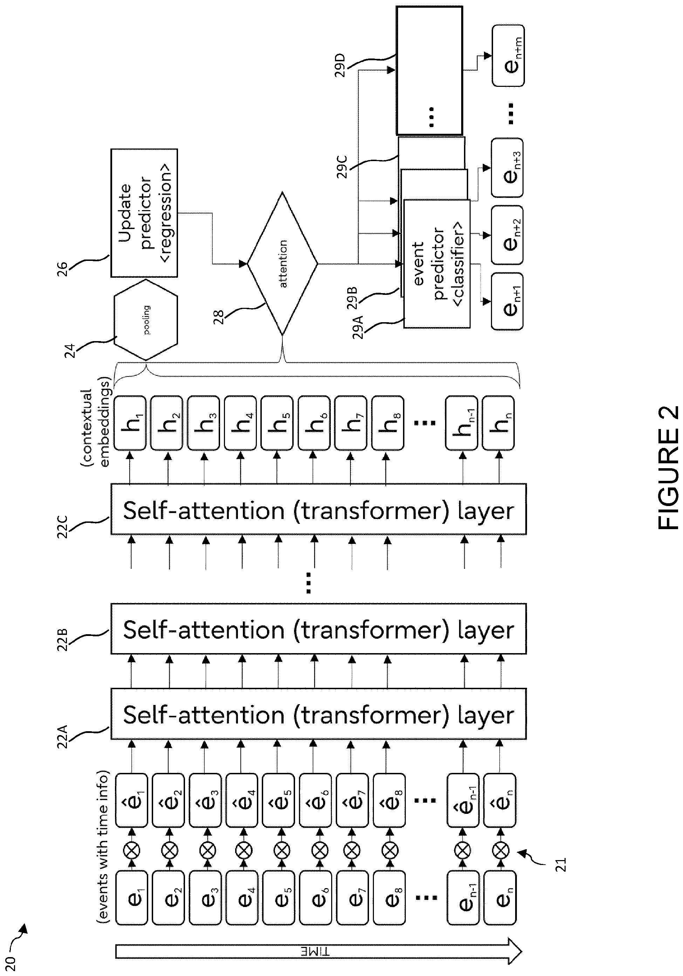

Figure 2 is a diagram illustrating the prognosis prediction model 20 according to a particular implementation.

Given some patient's clinical data and a proposed treatment, the model 20 predicts the outcome (future clinical data) of the same patient after they went through the input treatment. The neural network-based implementation of the prognosis prediction model 20 is able to learn from unlabelled clinical data. Relevant clinical information (e.g. diagnoses, symptoms, drugs, procedures, etc.) in encoded as timed events. The input treatment is encoded as part of the timed event set (as if it had been administered).

In particular, the model 20 makes its prediction based on medical history information relating to the target patient, which comprises medical history information from records or a database (e.g. from the EHR) together with a proposed treatment. The proposed treatment is included in the medical history information as if it had already occurred.

The medical history information is input to the model 20 as a series of events "e" with time information. Each event "e" is augmented with the time information to generate augmented event information "e-hat". This augmentation is referred to herein as time embedding/encoding and may be considered analogous to positional embedding, in which each word in a sentence is encoded with information representing its position in the sentence, except instead of position, time (e.g. in the form of a number of days elapsed since the first event) is used. The time embedding may be implemented in a number of ways, for example using a sine function and a cosine function to encode the time of an event into a vector and adding the vector on top of the event information "e". Time-Embeddings in time series representations may be used in transformer-based architectures, which generally use a positional encoding module. While some use the original fixed sinusoidal positional encoding, others may learn a time-embedding using a linear layer and a fully-connected layer respectively. An example of time-embedding for time series is Time2Vec (Kazemi et al., "Time2Vec for Time Series features encoding", 2019, https://arxiv.org/abs/1907.05321), which was developed for supervised learning tasks. The time embedding function is indicated by reference sign 21 in Figure 2.

A series of self-attention layers 22A, 22B, 22C (referred to together as 22) generate contextualized embeddings ("h") based on the augmented event information e-hat. The self-attention layers 22 are for example similar to those used in conventional transformer architectures.

The self-attention layers 22 (and optionally also the time embedding function 21) may be considered a first encoder model. The first encoder model encodes the medical history information relating to the target patient to generate target contextualized embeddings h.

The prognosis prediction model 20 further comprises a pooling layer 24, an update predictor 26, an attention network 28, and a plurality of event predictors 29 (29A, 29B, 29C, 29D). The pooling layer 24, update predictor 26, attention network 28, and plurality of event predictors 29 may be considered a prediction model. That is, the prognosis prediction model 20 may be considered to comprise a first encoder model and a prediction model as set out above.

The pooling layer 24 pools the contextualized embeddings to generate a single output vector for input to the update predictor 26 (many pooling techniques may be used here; an example which may be advantageous is self-attention pooling). The update predictor 26 uses the output of the pooling layer 24 to predict a time of at least one next event in the series of events e that makes up the medical history information related to the target patient. The update predictor in a specific implementation comprises a neural network and uses regression techniques to predict the time of the next event.

The attention network 28 uses the contextualized embeddings h and the output of the update predictor 26 to generate a vector that summarises the information contained in the embeddings h at the time predicted by the update predictor 26. This vector is output to the plurality of event predictors 29. Each predictor 29 predicts a single event (probability for presence/absence).

The plurality of event predictors 29 predict at least one next event. For example, in a specific implementation each event predictor is a classifier (neural network) which predicts whether an item of medical history information (an event) is positive or negative (or absent). For example, a classifier 29 may predict whether the presence of a symptom is positive (present in the target patient) or negative (determined to be not present) or absent not determined to be either present or absent), and/or another classifier 29 may predict whether a diagnosis of a disease or condition is positive or negative of absent. In this way, at least one next event is predicted by the prognosis prediction model 20 (together with the time of the at least one next event) assuming that the proposed treatment had already been administered to the target patient - in other words, at least on prognosis is predicted.

Although specific numbers of self-attention layers 22 and event predictors 29 have been given reference signs in Figure 2, the prognosis prediction model 20 may include any number of self-attention layers 20 and any number of event predictors 29. The specific implementation of the prognosis prediction model 20 illustrated in Figure 2 and described above is not essential and other implementations may be used. For example, other machine learning or deep learning methods/architectures could be used such as recurrent neural networks (RNN) or convolutional neural networks (CNN).

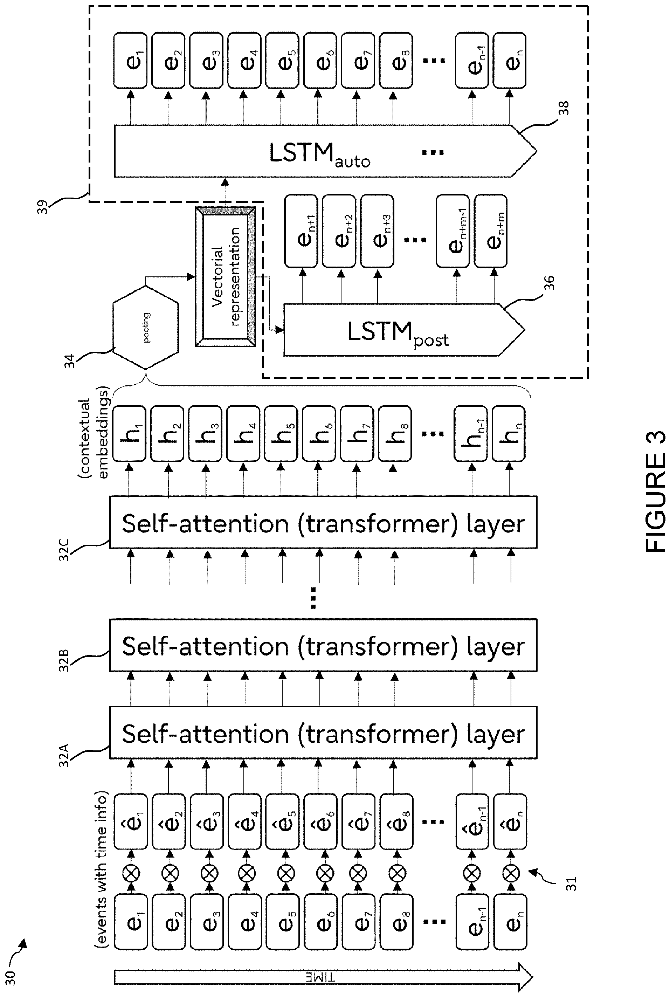

Figure 3 is a diagram illustrating an implementation of the patient similarity model 30.

The patient similarity model 20 uses vector distance (such as cosine similarity) to determine the similarity between instances of medical history information among medical history information relating to a number of subjects. A vectorial representation is generated for each medical history instance, and a vectorial representation is generated for modified medical history information relating to the target patient, the modified medical history information comprising the medical history information relating to the target patient together with the at least one predicted prognosis (predicted by the prognosis prediction model 20).

A medical history instance is a series of events relating to a given subject. It is not necessarily the case that each subject among the available data gives rise to only one medical history instance - for example, if the available data includes n events relating to a given subject, one medical history instance may include the n events, a further medical history instance may include n-1 of those events (e.g. all but the last one), a further medical history instance may include n-2 of those events (e.g. all but the last two or first two, or all but the first one and last one), etc. For example, a medical history instance comprises a series of consecutive events relating to a given subject.

The generation of a vectorial representation of a single medical history instance is illustrated in Figure 3. The same process is carried out for a plurality of medical history instances (and for the modified medical history information relating to the target patient). The elements of the dotted L-shaped box 39 are elements used for training the patient similarity model 30 and are described later.

The patient similarity model 30 comprises a time embedding function 31, a series of self-attention layers 32, and a pooling layer 34. The function of these elements is the same as that of the corresponding elements in the prognosis prediction model 20 described above with reference to Figure 2 and duplicate description is omitted. The elements 31, 32, and 34 may be referred to as a second encoder model. The architecture of the second encoder model in this implementation is the same as that of the first encoder model. However, while the first encoder network (used for prognosis prediction) is optimized during training to generalize well avoiding overfitting, the second encoder model is optimized during training to compress the clinical information into a small vector.

Although not shown in Figure 3, the patient similarity model 30 comprises a comparison function to compare the vectorial representation of the modified medical history information relating to the target patient with a plurality of vectorial representations of medical history instances to find at least one most similar medical history instance. The comparison may comprise computing a cosine distance and/or another distance measure between the vectorial representations.

The data from which the medical history instances are drawn may be the same as training data used to train the prognosis prediction model (and the patient similarity model).

Training of the prognosis prediction model 20 is carried out by using the prognosis prediction model 20 to predict, for a given subject, a prognosis which is already known based on the training data, and then weights of the elements in the prognosis prediction model 20 are updated based on results of a comparison between the predicted prognosis and the known prognosis. This is performed for a predetermined number of iterations and/or until an error threshold is met (based on the error between predicted and ground truth prognoses). The training may include predicting next events in general rather than only predicting prognoses (events following a treatment).

Training of the patient similarity model 20, or more particularly the second encoder model is carried out using the elements in the L-shaped box 39 in Figure 3. Following the generation of a vectorial representation of a medical history instance from among the training data, a decoder model 38 is used to generate a reconstructed medical history instance (in the form of a series of events e as illustrated), and weights of the second encoder model are adjusted based on the results of a comparison between the reconstructed medical history instance and the actual (ground truth) medical history instance. The decoder model 38 in a specific implementation comprises a long short-term memory (LSTM network).

In some implementations, the training of the second encoder model includes use of an auxiliary decoder model 36 which generates, based on a vectorial representation, an auxiliary reconstructed medical history instance comprising additionally at least one predicted next event. This auxiliary reconstructed medical history instance is compared with an auxiliary medical history instance from among the plurality of medical history instances comprising the events of the medical history instance and at least one next event. In this case the adjustment of the weights of the second encoder model includes adjustment based on this comparison (auxiliary comparison). The auxiliary decoder model 36 in a specific implementation comprises a long short-term memory (LSTM network).

When training the prognosis prediction model 20 the first encoder model may be initialized using the weights of the second encoder model after training of the second encoder model (but not necessarily with those weights frozen). In other words, an initial form of the first encoder model before training of the prognosis prediction model 20 may be the same as the second encoder model after training of the second encoder model. The use of the auxiliary decoder model 36 may help train the second encoder model to generate vectorial representations in a way conducive to prediction of next events, which may be useful if for example the weights of the first encoder are initialized using the weights of the second encoder model after training.

The pooling layers 24 and 34 may use a weighted combination of the contextualized embeddings and the weights concerned may be adjusted during training of the models 20 and 30.

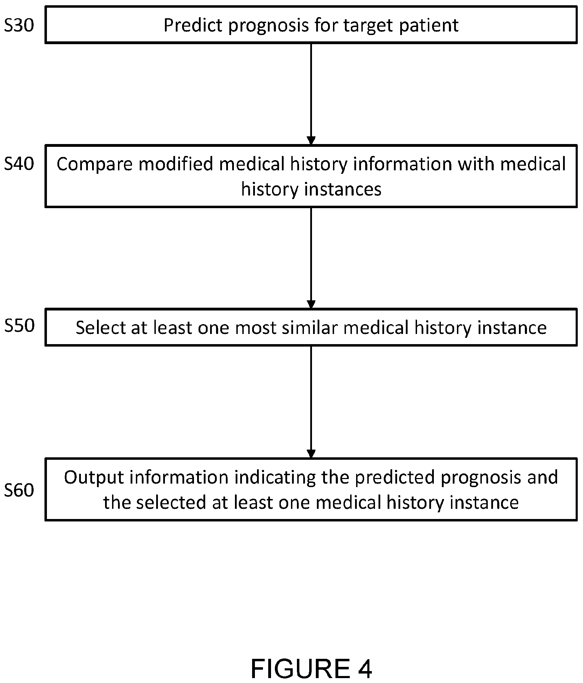

Figure 4 is a flowchart illustrating a method disclosed herein. The method comprises steps S30, S40, S50, and S60.

Step S30 comprises predicting a prognosis for a target patient. That is, step S30 comprises using an ML model to predict a prognosis for a target patient based on medical history information relating to the target patient, the medical history information relating to the target patient including an indication of a (proposed) treatment to be given to the target patient, wherein the ML model has been trained on training data comprising medical history information relating to a plurality of subjects. The ML model referred to herein may be considered the prognosis prediction model described above with reference to Figure 2, and any description therein may apply here and vice versa.

Step S40 comprises comparing modified medical history information with medical history instances. That is, step S40 comprises comparing modified medical history information relating to the target patient, comprising the medical history information relating to the target patient and the predicted prognosis, with a plurality of medical history instances, each medical history instance comprising medical history information from among the training data and relating to a subject among the plurality of subjects (and to a time period). The comparison may include use of the patient similarity model 30 described above with reference to Figure 3, and any description therein may apply here and vice versa.

Step S50 comprises selecting at least one most similar medical history instance. That is, step S50 comprises selecting based on the comparison at least one medical history instance most similar to the modified medical history information relating to the target patient.

Step S60 comprises outputting information indicating the predicted prognosis and the selected at least one medical history instance.

In line with Figure 4, for example, and according to a first embodiment there is disclosed herein a computer-implemented method comprising: using an ML model to predict a prognosis for a target patient based on medical history information relating to the target patient, the medical history information relating to the target patient including an indication of a (proposed) treatment to be given to the target patient, wherein the ML model has been trained on training data comprising medical history information relating to a plurality of subjects; comparing modified medical history information relating to the target patient, comprising the medical history information relating to the target patient and the predicted prognosis, with a plurality of medical history instances, each medical history instance comprising medical history information from among the training data and relating to a subject among the plurality of subjects (and to a time period); based on the comparison, selecting at least one medical history instance most similar to the modified medical history information relating to the target patient; and outputting information indicating the predicted prognosis and the selected at least one medical history instance.

The ML model may comprise: a first encoder model to encode the medical history information relating to the target patient to generate target contextualized embeddings; and a prediction model to predict, based on the target contextualized embeddings, the prognosis.

Comparing the modified medical history information relating to the target patient with the plurality of medical history instances may comprise: using a second encoder model to encode each medical history instance to generate contextualized embeddings of each medical history instance; generating a vectorial representation of each medical history instance based on the contextualized embeddings of the medical history instance; using the second encoder model to encode the modified medical history information relating to the target patient to generate contextualized embeddings of the modified medical history information relating to the target patient; generating a vectorial representation of the modified medical history information relating to the target patient based on the contextualized embeddings of the modified medical history information relating to the target patient; and comparing the vectorial representation of the modified medical history information relating to the target patient with the vectorial representations of the medical history instances.

Comparing the vectorial representation of the modified medical history information relating to the target patient with the vectorial representations of the medical history instances may comprise computing, for each of the vectorial representations of the medical history instance, a cosine distance between the vectorial representation of the modified medical history information relating to the target patient and the vectorial representation of the medical history instance.

The first encoder model may comprise a plurality of self-attention layers.

The second encoder model may comprise a plurality of self-attention layers.

The first encoder model may comprise a first plurality of self-attention layers and the second encoder model may comprise a second plurality of self-attention layers.

Encoding the medical history information relating to the target patient may comprise augmenting information relating to each event in the medical history information relating to the target patient with time information of the event and generating the target contextualized embeddings corresponding to each event by applying the self-attention layers to the augmented information.

The prediction model may comprise a first neural network to predict, based on the target contextualized embeddings, an event time of the predicted prognosis, and a second neural network to predict, based on the target contextualized embeddings and the predicted event time, the prognosis.

The second neural network may comprise a plurality of classifiers corresponding to a plurality of possible events/diagnoses.

The prediction model may comprise an attention network to generate a summary vector based on the target contextualized embeddings and based on the event time predicted by the first neural network.

The predicting by the second neural network, based on the target contextualized embeddings and the predicted event time, the prognosis may comprise predicting, based on the summary vector, the prognosis.

The prediction model may comprise an attention network to generate a summary vector based on the target contextualized embeddings and based on the event time predicted by the first neural network and the predicting by the second neural network, based on the target contextualized embeddings and the predicted event time, the prognosis may comprise predicting, based on the summary vector, the prognosis.

The first encoder model may be the same as the second encoder model.

The medical history information relating to the target patient may comprise a plurality/series of events.

The medical history information relating to the plurality of subjects may comprise a plurality/series of events relating to each of the plurality of subjects.

The medical history information relating to the target patient may comprise information indicating any of the following relating to the target patient: at least one diagnosis; at least one treatment; at least one report of at least one symptom; at least one physiological measurement; at least one operation and/or surgery; at least one administered therapy; at least one medical procedure; a sex assigned at birth; an ethnicity; at least one (germline) mutation.

The medical history information relating to the plurality of subjects may comprise, for each of the plurality of subjects, information indicating any of the following relating to the subject: at least one diagnosis; at least one treatment; at least one report of at least one symptom; at least one physiological measurement; at least one operation and/or surgery; at least one administered therapy; at least one medical procedure; a sex assigned at birth; an ethnicity; at least one (germline) mutation.

The medical history information relating to the target patient may comprise information from an electronic heath record relating to the target patient.

The medical history information relating to the plurality of subjects may comprise, for each of the plurality of subjects, information from an electronic heath record relating to the subject.

The medical history information relating to the target patient may comprise time series data.

The medical history information relating to the plurality of subjects may comprise time series data.

The computer-implemented method may comprise outputting information indicating the medical history information relating to the subject corresponding to the selected at least one medical history instance.

The predicted prognosis may comprise a predicted diagnosis of a condition or disease.

The computer-implemented method may comprise: using the ML model to predict a further prognosis for the target patient based on further medical history information relating to the target patient, the further medical history information relating to the target patient including an indication of a further (proposed) treatment to be given to the target patient; comparing further modified medical history information relating to the target patient, comprising the further medical history information relating to the target patient and the further predicted prognosis, with the plurality of medical history instances; based on the comparison, selecting a further at least one medical history instance most similar to the further modified medical history information relating to the target patient; and outputting further information indicating the further predicted prognosis and the selected further at least one medical history instance.

The computer-implemented method may comprise: using the ML model to predict a first prognosis for the target patient based on first medical history information relating to the target patient, the first medical history information relating to the target patient including an indication of a first (proposed) treatment to be given to the target patient; comparing first modified medical history information relating to the target patient, comprising the first medical history information relating to the target patient and the first predicted prognosis, with the plurality of medical history instances; based on the comparison, selecting a first at least one medical history instance most similar to the first modified medical history information relating to the target patient; using the ML model to predict a second prognosis for the target patient based on second medical history information relating to the target patient, the second medical history information relating to the target patient including an indication of a second (proposed) treatment to be given to the target patient; comparing second modified medical history information relating to the target patient, comprising the second medical history information relating to the target patient and the second predicted prognosis, with the plurality of medical history instances; based on the comparison, selecting a second at least one medical history instance most similar to the second modified medical history information relating to the target patient; and outputting first information indicating the first predicted prognosis and the selected first at least one medical history instance and second information indicating the second predicted prognosis and the selected second at least one medical history instance.

The second encoder model may have been trained according to a second encoder model training process comprising: using the second encoder model to encode a medical history instance to generate contextualized embeddings of the medical history instance; generating a vectorial representation of the medical history instance based on the contextualized embeddings of the medical history instance; using a decoder model to generate a reconstructed medical history instance based on the vectorial representation; comparing the medical history instance with the reconstructed medical history instance; and adjusting at least one weight of the second encoder model based on the comparison.

The second encoder model training process may comprise iterating, for a plurality of the medical history instances, the steps of: using the second encoder model to encode a medical history instance to generate contextualized embeddings of the medical history instance; generating a vectorial representation of the medical history instance based on the contextualized embeddings of the medical history instance; using a decoder model to generate a reconstructed medical history instance based on the vectorial representation; comparing the reconstructed medical history instance with the medical history instance; and adjusting at least one weight of the second encoder model based on the comparison.

The second encoder model training process may comprise iterating the steps for a predetermined number of iterations and/or until an error threshold is met.

The second encoder model training process may further comprise: using an auxiliary decoder model to generate an auxiliary reconstructed medical history instance comprising at least one predicted event; and comparing the auxiliary reconstructed medical history instance with an auxiliary medical history instance from among the plurality of medical history instances comprising the events of the medical history instance and at least one next event, wherein adjusting at least one weight of the second encoder model comprises adjusting at least one weight of the second encoder model based on the comparison of the auxiliary reconstructed medical history instance with the auxiliary medical history instance.

The decoder model may comprise a recurrent neural network.

The auxiliary decoder model may comprise a recurrent neural network.

The decoder model may comprise a long short-term memory, LSTM, network.

The auxiliary decoder model may comprise an LSTM network.

The ML model may have been trained according to an ML model training process comprising: using the ML model to predict a prognosis for a subject among the plurality of subjects based on training data comprising medical history information relating to the subject, the medical history information relating to the subject including an indication of a treatment given to the subject; comparing the predicted prognosis with a ground truth prognosis in the training data; and adjusting at least one weight of the ML model based on the comparison.

The ML model training process may comprise iterating, for a plurality of the subjects, the steps of: using the ML model to predict a prognosis for a subject among the plurality of subjects based on training data comprising medical history information relating to the subject, the medical history information relating to the subject including an indication of a treatment given to the subject; comparing the predicted prognosis with a ground truth prognosis in the training data; and adjusting at least one weight of the ML model based on the comparison.

The ML model training process may comprise iterating the steps for a predetermined number of iterations and/or until an error threshold is met.

The computer-implemented method may comprise (before predicting the prognosis) training the ML model according to the ML model training process.

The computer-implemented method may comprise (before comparing the modified medical history information relating to the target patient with the plurality of medical history instances) training the second encoder model according to the second encoder model training process.

The computer-implemented method may comprise training the second encoder model before training the ML model, wherein training the ML model according to the ML model training process comprises initializing the weights of the first encoder model using the weights of the second encoder model (after training the second encoder model).

The computer-implemented method may comprise: (before predicting the prognosis) training the ML model according to the ML model training process; and (before comparing the modified medical history information relating to the target patient with the plurality of medical history instances) training the second encoder model according to the second encoder model training process, wherein the computer-implemented method comprises training the second encoder model before training the ML model, wherein training the ML model according to the ML model training process comprises initializing the weights of the first encoder model using the weights of the second encoder model (after training the second encoder model).

The second encoder model may have been trained according to a second encoder model training process comprising: using the second encoder model to encode a medical history instance to generate contextualized embeddings of the medical history instance; generating a vectorial representation of the medical history instance based on the contextualized embeddings of the medical history instance; using a decoder model to generate a reconstructed medical history instance based on the vectorial representation; comparing the medical history instance with the reconstructed medical history instance; and adjusting at least one weight of the second encoder model based on the comparison, and wherein the ML model has been trained according to an ML model training process comprising: using the ML model to predict a prognosis for a subject among the plurality of subjects based on training data comprising medical history information relating to the subject, the medical history information relating to the subject including an indication of a treatment given to the subject; comparing the predicted prognosis with a ground truth prognosis in the training data; and adjusting at least one weight of the ML model based on the comparison.

The computer-implemented method may comprise training the second encoder model according to the second encoder model training process and, after training the second encoder model, training the ML model according to the ML model training process, wherein training the ML model according to the ML model training process comprises initializing the weights of the first encoder model using the weights of the second encoder model (after training the second encoder model).

Outputting the information may comprise outputting the information on a display.

The treatment may comprise any of a drug, a therapy, and a medical procedure.

The computer-implemented method may comprise: using a first at least one processor and/or computing device to encode each medical history instance using the second encoder model, generate the vectorial representation of each medical history instance, encode the modified medical history information using the second encoder model, and generate the vectorial representation of the modified medical history information; and using a second at least one processor and/or computing device to compare the vectorial representation of the modified medical history information relating to the target patient with the vectorial representations of the medical history instances, wherein the first at least one processor and/or computing device has a larger processing power and/or computing load than the second at least one processor and/or computing device.

The use of the first at least one processor and/or computing device may be partially simultaneous with the use of the second at least one processor and/or computing device.

According to a second embodiment there is disclosed herein a computer program which, when run on a computer, causes the computer to carry out a method comprising: using a machine learning, ML, model to predict a prognosis for a target patient based on medical history information relating to the target patient, the medical history information relating to the target patient including an indication of a (proposed) treatment to be given to the target patient, wherein the ML model has been trained on training data comprising medical history information relating to a plurality of subjects; comparing modified medical history information relating to the target patient, comprising the medical history information relating to the target patient and the predicted prognosis, with a plurality of medical history instances, each medical history instance comprising medical history information from among the training data and relating to a subject among the plurality of subjects (and to a time period); based on the comparison, selecting at least one medical history instance most similar to the modified medical history information relating to the target patient; and outputting information indicating the predicted prognosis and the selected at least one medical history instance.

According to a third embodiment there is disclosed herein an information processing apparatus comprising a memory and a processor connected to the memory, wherein the processor is configured to: use a machine learning, ML, model to predict a prognosis for a target patient based on medical history information relating to the target patient, the medical history information relating to the target patient including an indication of a (proposed) treatment to be given to the target patient, wherein the ML model has been trained on training data comprising medical history information relating to a plurality of subjects; compare modified medical history information relating to the target patient, comprising the medical history information relating to the target patient and the predicted prognosis, with a plurality of medical history instances, each medical history instance comprising medical history information from among the training data and relating to a subject among the plurality of subjects (and to a time period); based on the comparison, select at least one medical history instance most similar to the modified medical history information relating to the target patient; and output information indicating the predicted prognosis and the selected at least one medical history instance.

According to a fourth embodiment there is disclosed herein a system comprising a plurality of computing devices configured to: use a machine learning, ML, model to predict a prognosis for a target patient based on medical history information relating to the target patient, the medical history information relating to the target patient including an indication of a (proposed) treatment to be given to the target patient, wherein the ML model has been trained on training data comprising medical history information relating to a plurality of subjects; compare modified medical history information relating to the target patient, comprising the medical history information relating to the target patient and the predicted prognosis, with a plurality of medical history instances, each medical history instance comprising medical history information from among the training data and relating to a subject among the plurality of subjects (and to a time period); based on the comparison, select at least one medical history instance most similar to the modified medical history information relating to the target patient; and output information indicating the predicted prognosis and the selected at least one medical history instance.

Comparing the modified medical history information relating to the target patient with the plurality of medical history instances may comprise: using a second encoder model to encode each medical history instance to generate contextualized embeddings of each medical history instance; generating a vectorial representation of each medical history instance based on the contextualized embeddings of the medical history instance; using the second encoder model to encode the modified medical history information relating to the target patient to generate contextualized embeddings of the modified medical history information relating to the target patient; generating a vectorial representation of the modified medical history information relating to the target patient based on the contextualized embeddings of the modified medical history information relating to the target patient; and comparing the vectorial representation of the modified medical history information relating to the target patient with the vectorial representations of the medical history instances, wherein the plurality of computing devices comprises: a first at least one processor or computing device configured to encode each medical history instance using the second encoder model, generate the vectorial representation of each medical history instance, encode the modified medical history information using the second encoder model, and generate the vectorial representation of the modified medical history information; and a second at least one processor or computing device configured to compare the vectorial representation of the modified medical history information relating to the target patient with the vectorial representations of the medical history instances, wherein the first at least one processor or computing device has a larger processing power and/or computing load than the second at least one processor or computing device.

The use of the first at least one processor or computing device may be partially simultaneous with the use of the second at least one processor or computing device.

Features relating to any aspect/embodiment may be applied to any other aspect/embodiment.

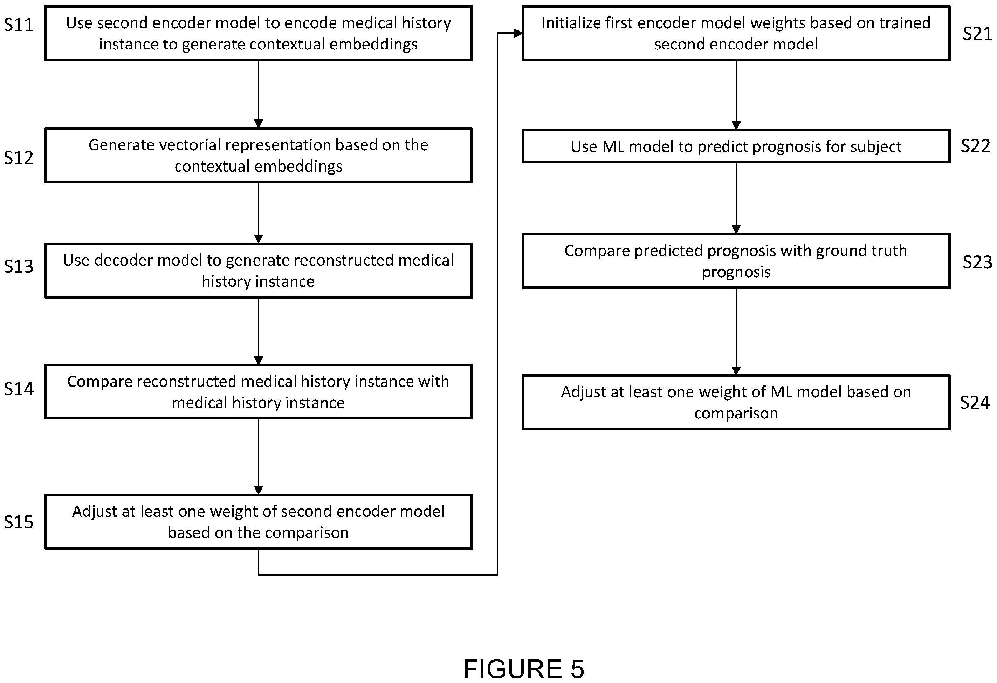

Figure 5 is a flowchart illustrating a training method which may be used to train the ML model and the second encoder model. The method comprises steps S11, S12, S13, S14, S15, S21, S22, S23, S24, and S25.

Step S11 comprises using the second encoder model to encode a medical history instance (from among training data) to generate contextualized embeddings. Step S12 comprises generating a vectorial representation of the medical history instance based on the contextualized embeddings. Step S13 comprises using a decoder model to generate a reconstructed medical history instance.

Step S14 comprises comparing the reconstructed medical history instance with the (original) medical history instance. Step S15 comprises adjusting at least one weight of the second encoder model based on the comparison.

Steps S11-S15 may be repeated (using different medical history instances from among the training data) for a given number of iterations and/or until an error threshold is met.

Step S21 comprises initializing the first encoder model weights based on the trained second encoder model, e.g. using the weights of the trained second encoder model. Step S22 comprises using the ML model to predict a prognosis for a subject (based on a treatment) using a medical history instance from the training data. Step S23 comprises comparing the predicted prognosis with a ground truth prognosis (i.e. the actual prognosis from the training data). Step S24 comprises adjusting at least one weight of the ML model based on the comparison.

Steps S22-S24 may be repeated (using different medical history instances from among the training data) for a given number of iterations and/or until an error threshold is met.

The Figure 5 training method is not essential and the ML model and second encoder network may be trained in other ways. It will be appreciated that steps of the training method illustrated in Figure 5 correspond to method steps described above, e.g. with respect to Figure 4, and description therein may apply here and vice versa. Steps S11-S15 may be considered a second encoder model training process and/or steps S22-S24 (or steps S21-S24) may be considered an ML model training process. A method disclosed herein comprises the Figure 5 method followed by the Figure 4 method.

### Worked Example

A worked example of using the prognosis prediction model 20 and patient similarity model 30 to predict a prognosis and find similar patients will be described. EHR data of the target patient (medical history information) may be represented in a JSON file, an example of which is shown below (not exhaustive).

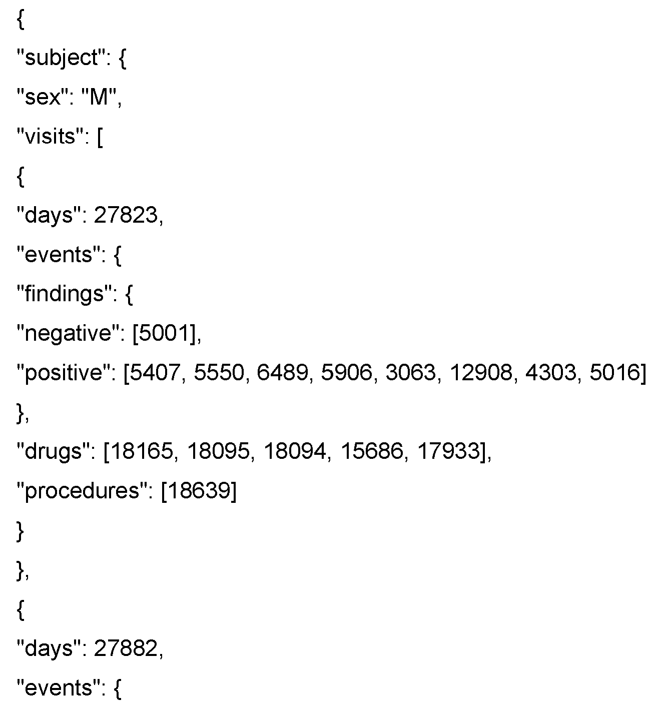

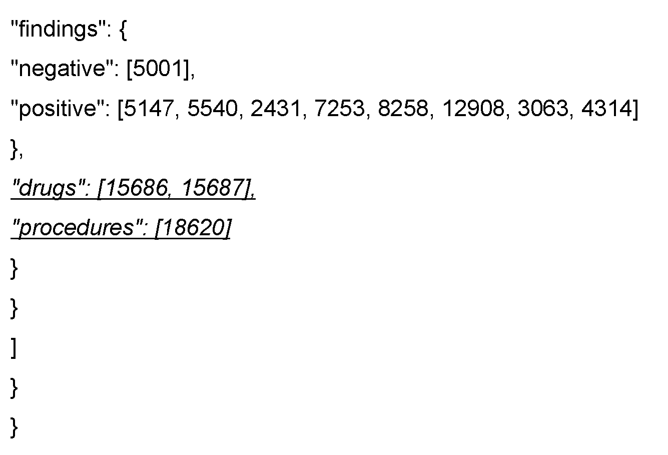

In this JSON example, some data about the target patient is shown that will be input to the models 20 and 30. A short example has been used for the sake of brevity. The data includes the sex assigned at birth under the key "sex". In practice, this information is included as an event with the time information indicating the time of birth (i.e. "0 days"). Similarly, other information, e.g. information about germline mutations or ethnicity, etc., could be included in the same fashion.

A sequence of event sets under the key "visits" is shown. This represents points in time when the patient visited a doctor or a hospital and some events were recorded; although it could also include some events recorded by a device or by the patient themselves. Each visit includes a "days" key. This key represents the number of days since the target patient's birth-date. In this example the target patient was 27823 days old, that is about 76 years and 3 months old, at the date of the first visit shown above. The next visit happens roughly 2 months later (59 days). The age of the patient is represented in days to avoid dates altogether. The "events" key contains all the events recorded at that date. Under "findings" is listed the diagnosed diseases and symptoms as "positive". The "negative" set contains the conditions that the doctor found to be absent. For example, if the patient was found or determined NOT to have fever that would be listed under "negative". The different events are referred to using IDs as recorded in an associated database. As an example, 12908 could represent "Acute on chronic combined systolic and diastolic heart failure". Treatments administered in the past are also recorded in the above under "drugs" and "procedures".

The last entry also contains a proposed treatment (insertion of a single chamber device and some drugs) underlined and in italics. The example shown above input to the system (prognosis prediction model 20 and patient similarity model 30) generates predicted prognoses and similar patient information according to the output shown below in JSON format.

```
 {"predictions": [
 { "days": 27899,
 "predicted": [[0.30, 7587]],
 " similar": [29301, 27399]
 },
 { "days": 27921,
 "predicted": [[0.48, 7587], [0.31, 7584]],
 "similar": [29301]
 }
 ]}
```

As shown above, the system produces predictions for dates in the future, as represented by "days". Each predicted event is represented by a pair (probability, id). For each predicted event date the predictions that were predicted with a probability above certain threshold (0.30 for the example) are considered. When a certain event goes above the threshold a new prediction set may be included. It is apparent from the above that the event 7587 increased in probability for later dates. The IDs of some patients that had a similar record are also recorded. The "similar" patients have not only a similar past record but also, for example, went through this treatment and recorded a response similar to the predicted one. As the prediction goes further into the future the confidence of the prediction may be lower and the number of recorded evidence (i.e. similar other patients) smaller.

In this worked example, an ID of a similar patient is output as information indicating the at least one most similar medical history instance. Alternatively or additionally, information specifically indicating the medical history instance may be output, for example an ID of a medical history instance (event sequence) that was most similar, or the event sequence itself.

By varying the proposed treatment (underlined and italicised in the input listing), different predictions and similar medical history instances may be generated and so the different treatments may be compared and the most appropriate/best treatment selected, e.g. by a clinician.

An aspect disclosed herein may be considered a Clinical Decision Support (CDS) System to help compare multiple candidate treatments if they were to be applied to a particular patient as described by their EHR. A useful feature is a mechanism to refer to evidence of the most similar known cases that had a similar treatment.

Aspects disclosed herein may aid in providing explainability of predicted prognoses by providing information indicating similar medical history instances/subjects. Since the predictions are not one-hundred-percent accurate, and since the prediction made by machine learning systems may affect the treatment administered to a patient, it may be useful for a clinician to confirm the utility of the predictions.

An objective/goal of aspects disclosed herein is a system that assists doctors in treatment decision by predicting the outcome of different candidate treatments supported by clinical evidence. Aspects disclosed herein go beyond providing a prediction for a prognosis by including a mechanism to provide also past clinical cases that correspond and support the queried patient's case. Aspects disclosed herein may include connection to a database of EHRs that will be used for both (1) training of the predictive model and (2) supporting evidence for predictions.

That is, due to the complexity of a prognosis prediction task, the predictions may not be guaranteed with large confidence margins, so the final decision of the treatment may remain with the clinician. Because of the responsibility of such a task, the clinician may naturally doubt the predictions of the system and will not prescribe a treatment they do not know well. Therefore, without providing evidence for the predictions (i.e. most similar medical history instances/subjects), the usability of such prognosis prediction may be limited. When deciding the best treatment for a patient in accordance with aspects disclosed herein, a clinician may study cases of similar subjects to improve the prognosis evaluation.

Aspects disclosed herein may be considered general (as in generally applicable to different treatments/outcomes), accurate and explainable at the same time.

Conventional methodologies for prognosis prediction may use past data of other subjects as training data and then predict a prognosis but thereafter this training data is discarded and the prediction is provided without explicit evidence or explanation. Aspects disclosed herein may use the training data also after training to augment output predictions with supporting evidence.

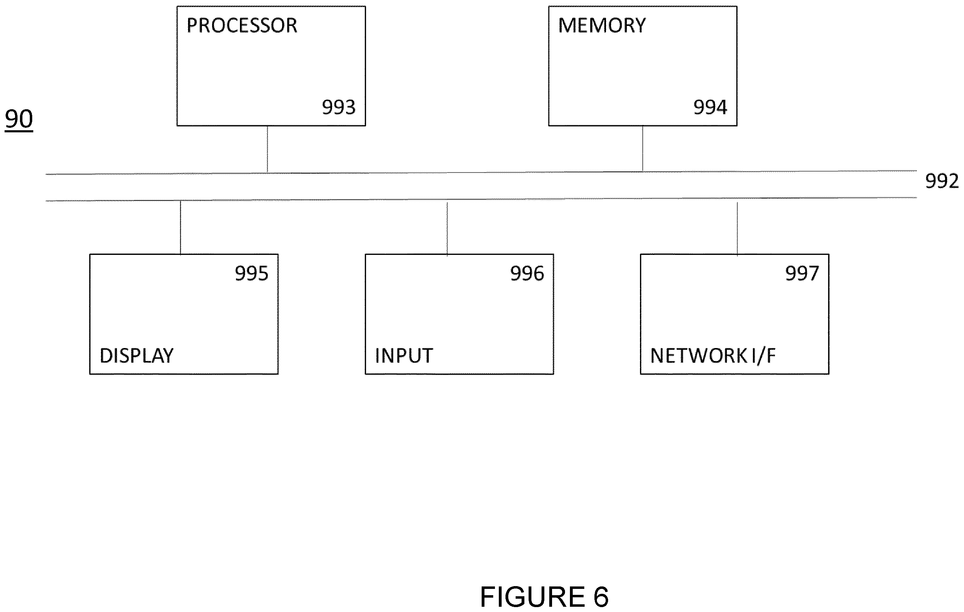

Figure 6 is a block diagram of an information processing apparatus 90 or a computing device 90, such as a data storage server, which embodies the present invention, and which may be used to implement some or all of the operations of a method embodying the present invention, and perform some or all of the tasks of apparatus of an embodiment. The computing device 90 may be used to implement any of the method steps described above, e.g. any of steps S2 and S3 and steps S30-S60 and steps S11-S24 and/or any operations of the models 20 and 30.

The computing device 90 comprises a processor 993 and memory 994. Optionally, the computing device also includes a network interface 997 for communication with other such computing devices, for example with other computing devices of invention embodiments. Optionally, the computing device also includes one or more input mechanisms such as keyboard and mouse 996, and a display unit such as one or more monitors 995. These elements may facilitate user interaction. The components are connectable to one another via a bus 992.

The memory 994 may include a computer readable medium, which term may refer to a single medium or multiple media (e.g., a centralized or distributed database and/or associated caches and servers) configured to carry computer-executable instructions. Computer-executable instructions may include, for example, instructions and data accessible by and causing a computer (e.g., one or more processors) to perform one or more functions or operations. For example, the computer-executable instructions may include those instructions for implementing a method disclosed herein, or any method steps disclosed herein, for example any of steps S2 and S3 and steps S30-S60 and steps S11-S24 and/or any operations of the models 20 and 30. Thus, the term "computer-readable storage medium" may also include any medium that is capable of storing, encoding or carrying a set of instructions for execution by the machine and that cause the machine to perform any one or more of the method steps of the present disclosure. The term "computer-readable storage medium" may accordingly be taken to include, but not be limited to, solid-state memories, optical media and magnetic media.

By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing computer program code stored in the memory 994 to implement any of the method steps described herein, for example any of steps S2 and S3 and steps S30-S60 and steps S11-S24 and/or any operations of the models 20 and 30. The memory 994 stores data being read and written by the processor 993 and may store medical history information and/or EHR data and/or proposed treatment information and/or information indicating at least one predicted prognosis and/or event information and/or augmented event information and/or contextualized embeddings and/or vectorial representations and/or reconstructed medical history instances and/or auxiliary reconstructed medical history instances and/or predictions and/or similarity information and/or any network weights described above and/or input data and/or other data, described above, and/or programs for executing any of the method steps described above. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and operations discussed herein. The processor 993 may be considered to comprise any of the modules described above, and e.g. any of models 20 and 30. Any operations described as being implemented by a module may be implemented as a method by a computer and e.g. by the processor 993.

The display unit 995 may display a representation of data stored by the computing device, such as a representation of medical history information and/or EHR data and/or proposed treatment information and/or information indicating at least one predicted prognosis and/or event information and/or augmented event information and/or contextualized embeddings and/or vectorial representations and/or reconstructed medical history instances and/o auxiliary reconstructed medical history instances and/or predictions and/or similarity information and/or any network weights described above and/or input data and/or other data, described above and/or GUI windows and/or interactive representations enabling a user to interact with the apparatus 90 by e.g. drag and drop or selection interaction, and/or any other output described above, and may also display a cursor and dialog boxes and screens enabling interaction between a user and the programs and data stored on the computing device. The display may be used to display information indicating at least one predicted prognosis and at least one most similar medical history instance. The input mechanisms 996 may enable a user to input data and instructions to the computing device, such as enabling a user to input any user input described above.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

Methods embodying the present invention may be carried out on a computing device/apparatus 90 such as that illustrated in Figure 6. Such a computing device need not have every component illustrated in Figure 6, and may be composed of a subset of those components. For example, the apparatus 90 may comprise the processor 993 and the memory 994 connected to the processor 993. Or the apparatus 90 may comprise the processor 993, the memory 994 connected to the processor 993, and the display 995. A method embodying the present invention may be carried out by a single computing device in communication with one or more data storage servers via a network. The computing device may be a data storage itself storing at least a portion of the data.

A method embodying the present invention may be carried out by a plurality of computing devices operating in cooperation with one another. One or more of the plurality of computing devices may be a data storage server storing at least a portion of the data. The device 90 may be a computing device among a plurality of similarly configured computing devices, wherein the computing devices are together configured for carrying out any method steps described herein, e.g. any of steps S2 and S3 and steps S30-S60 and steps S11-S24 and/or any operations of the models 20 and 30. For example, at least one said computing device may encode each medical history instance using the second encoder model, generate the vectorial representation of each medical history instance, encode the modified medical history information using the second encoder model, and generate the vectorial representation of the modified medical history information, and at least one other said computing device may compare the vectorial representation of the modified medical history information relating to the target patient with the vectorial representations of the medical history instances. The processors and/or computing devices may be heterogeneous e.g. in terms of processing power.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention (e.g. any of steps S2 and S3 and steps S30-S60 and steps S11-S24 and/or any operations of the models 20 and 30) may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The above-described embodiments of the present invention may advantageously be used independently of any other of the embodiments or in any feasible combination with one or more others of the embodiments.

## Claims

1. A computer-implemented method comprising:
using a machine learning, ML, model to predict a prognosis for a target patient based on medical history information relating to the target patient, the medical history information relating to the target patient including an indication of a treatment to be given to the target patient, wherein the ML model has been trained on training data comprising medical history information relating to a plurality of subjects;
comparing modified medical history information relating to the target patient, comprising the medical history information relating to the target patient and the predicted prognosis, with a plurality of medical history instances, each medical history instance comprising medical history information from among the training data and relating to a subject among the plurality of subjects;
based on the comparison, selecting at least one medical history instance most similar to the modified medical history information relating to the target patient; and
outputting information indicating the predicted prognosis and the selected at least one medical history instance.

2. The computer-implemented method as claimed in claim 1, wherein the ML model comprises:
a first encoder model to encode the medical history information relating to the target patient to generate target contextualized embeddings; and
a prediction model to predict, based on the target contextualized embeddings, the prognosis.

3. The computer-implemented method as claimed in claim 2, wherein the prediction model comprises a first neural network to predict, based on the target contextualized embeddings, an event time of the predicted prognosis, and a second neural network to predict, based on the target contextualized embeddings and the predicted event time, the prognosis.

4. The computer-implemented method as claimed in claim 3, wherein the second neural network comprises a plurality of classifiers corresponding to a plurality of possible events.

5. The computer-implemented method as claimed in claim 4, wherein the prediction model comprises an attention network to generate a summary vector based on the target contextualized embeddings and based on the event time predicted by the first neural network and wherein the predicting by the second neural network, based on the target contextualized embeddings and the predicted event time, the prognosis comprises predicting, based on the summary vector, the prognosis.

6. The computer-implemented method as claimed in any of claims 2 to 5, wherein the first encoder model comprises a plurality of self-attention layers.

7. The computer-implemented method as claimed in any of the preceding claims, wherein comparing the modified medical history information relating to the target patient with the plurality of medical history instances comprises:
using a second encoder model to encode each medical history instance to generate contextualized embeddings of each medical history instance;
generating a vectorial representation of each medical history instance based on the contextualized embeddings of the medical history instance;
using the second encoder model to encode the modified medical history information relating to the target patient to generate contextualized embeddings of the modified medical history information relating to the target patient;
generating a vectorial representation of the modified medical history information relating to the target patient based on the contextualized embeddings of the modified medical history information relating to the target patient; and
comparing the vectorial representation of the modified medical history information relating to the target patient with the vectorial representations of the medical history instances.

8. The computer-implemented method as claimed in claim 7, wherein the second encoder model comprises a plurality of self-attention layers.

9. The computer-implemented method as claimed in claim 7 or claim 8, wherein the second encoder model has been trained according to a second encoder model training process comprising:
using the second encoder model to encode a medical history instance to generate contextualized embeddings of the medical history instance;
generating a vectorial representation of the medical history instance based on the contextualized embeddings of the medical history instance;
using a decoder model to generate a reconstructed medical history instance based on the vectorial representation;
comparing the medical history instance with the reconstructed medical history instance; and
adjusting at least one weight of the second encoder model based on the comparison,
and wherein the ML model has been trained according to an ML model training process comprising:
using the ML model to predict a prognosis for a subject among the plurality of subjects based on training data comprising medical history information relating to the subject, the medical history information relating to the subject including an indication of a treatment given to the subject;
comparing the predicted prognosis with a ground truth prognosis in the training data; and
adjusting at least one weight of the ML model based on the comparison.

10. The computer-implemented method as claimed in claim 9, comprising training the second encoder model according to the second encoder model training process and, after training the second encoder model, training the ML model according to the ML model training process, wherein training the ML model according to the ML model training process comprises initializing the weights of the first encoder model using the weights of the second encoder model.

11. The computer-implemented method as claimed in any of claims 7 to 10, comprising:
using a first at least one processor and/or computing device to encode each medical history instance using the second encoder model, generate the vectorial representation of each medical history instance, encode the modified medical history information using the second encoder model, and generate the vectorial representation of the modified medical history information; and
using a second at least one processor and/or computing device to compare the vectorial representation of the modified medical history information relating to the target patient with the vectorial representations of the medical history instances,
wherein the first at least one processor and/or computing device has a larger processing power and/or computing load than the second at least one processor and/or computing device.

12. A computer program which, when run on a computer, causes the computer to carry out a method comprising:
using a machine learning, ML, model to predict a prognosis for a target patient based on medical history information relating to the target patient, the medical history information relating to the target patient including an indication of a treatment to be given to the target patient, wherein the ML model has been trained on training data comprising medical history information relating to a plurality of subjects;
comparing modified medical history information relating to the target patient, comprising the medical history information relating to the target patient and the predicted prognosis, with a plurality of medical history instances, each medical history instance comprising medical history information from among the training data and relating to a subject among the plurality of subjects;
based on the comparison, selecting at least one medical history instance most similar to the modified medical history information relating to the target patient; and
outputting information indicating the predicted prognosis and the selected at least one medical history instance.

13. An information processing apparatus comprising a memory and a processor connected to the memory, wherein the processor is configured to:
use a machine learning, ML, model to predict a prognosis for a target patient based on medical history information relating to the target patient, the medical history information relating to the target patient including an indication of a treatment to be given to the target patient, wherein the ML model has been trained on training data comprising medical history information relating to a plurality of subjects;
compare modified medical history information relating to the target patient, comprising the medical history information relating to the target patient and the predicted prognosis, with a plurality of medical history instances, each medical history instance comprising medical history information from among the training data and relating to a subject among the plurality of subjects;
based on the comparison, select at least one medical history instance most similar to the modified medical history information relating to the target patient; and
output information indicating the predicted prognosis and the selected at least one medical history instance.

14. A system comprising a plurality of computing devices configured to:
use a machine learning, ML, model to predict a prognosis for a target patient based on medical history information relating to the target patient, the medical history information relating to the target patient including an indication of a treatment to be given to the target patient, wherein the ML model has been trained on training data comprising medical history information relating to a plurality of subjects;
compare modified medical history information relating to the target patient, comprising the medical history information relating to the target patient and the predicted prognosis, with a plurality of medical history instances, each medical history instance comprising medical history information from among the training data and relating to a subject among the plurality of subjects;
based on the comparison, select at least one medical history instance most similar to the modified medical history information relating to the target patient; and
output information indicating the predicted prognosis and the selected at least one medical history instance.

15. The system as claimed in claim 14, wherein comparing the modified medical history information relating to the target patient with the plurality of medical history instances comprises:
using a second encoder model to encode each medical history instance to generate contextualized embeddings of each medical history instance;
generating a vectorial representation of each medical history instance based on the contextualized embeddings of the medical history instance;
using the second encoder model to encode the modified medical history information relating to the target patient to generate contextualized embeddings of the modified medical history information relating to the target patient;
generating a vectorial representation of the modified medical history information relating to the target patient based on the contextualized embeddings of the modified medical history information relating to the target patient; and
comparing the vectorial representation of the modified medical history information relating to the target patient with the vectorial representations of the medical history instances,
wherein the plurality of computing devices comprises:
a first at least one processor or computing device configured to encode each medical history instance using the second encoder model, generate the vectorial representation of each medical history instance, encode the modified medical history information using the second encoder model, and generate the vectorial representation of the modified medical history information; and
a second at least one processor or computing device configured to compare the vectorial representation of the modified medical history information relating to the target patient with the vectorial representations of the medical history instances,
wherein the first at least one processor or computing device has a larger processing power and/or computing load than the second at least one processor or computing device.

## Amended claims

### Amended claims in accordance with Rule 137(2) EPC.

1. A computer-implemented method comprising:
using a machine learning, ML, model to predict (S30) a prognosis for a target patient based on medical history information relating to the target patient, the medical history information relating to the target patient including an indication of a treatment to be given to the target patient, wherein the ML model has been trained on training data comprising medical history information relating to a plurality of subjects;
comparing (S40) modified medical history information relating to the target patient, comprising the medical history information relating to the target patient and the predicted prognosis, with a plurality of medical history instances, each medical history instance comprising medical history information from among the training data and relating to a subject among the plurality of subjects;
based on the comparison, selecting (S50) at least one medical history instance most similar to the modified medical history information relating to the target patient; and
outputting (S60) information indicating the predicted prognosis and the selected at least one medical history instance,
wherein comparing (S40) the modified medical history information relating to the target patient with the plurality of medical history instances comprises:
using a second encoder model to encode each medical history instance to generate contextualized embeddings of each medical history instance;
generating a vectorial representation of each medical history instance based on the contextualized embeddings of the medical history instance;
using the second encoder model to encode the modified medical history information relating to the target patient to generate contextualized embeddings of the modified medical history information relating to the target patient;
generating a vectorial representation of the modified medical history information relating to the target patient based on the contextualized embeddings of the modified medical history information relating to the target patient; and
comparing the vectorial representation of the modified medical history information relating to the target patient with the vectorial representations of the medical history instances,
wherein the computer-implemented method comprises:
using a first at least one processor and/or computing device to encode each medical history instance using the second encoder model, generate the vectorial representation of each medical history instance, encode the modified medical history information using the second encoder model, and generate the vectorial representation of the modified medical history information; and
using a second at least one processor and/or computing device to compare the vectorial representation of the modified medical history information relating to the target patient with the vectorial representations of the medical history instances,
wherein the first at least one processor and/or computing device has a larger processing power and/or computing load than the second at least one processor and/or computing device, and wherein the use of the first at least one processor and/or computing device is partially simultaneous with the use of the second at least one processor and/or computing device.

2. The computer-implemented method as claimed in claim 1, wherein the ML model comprises:
a first encoder model to encode the medical history information relating to the target patient to generate target contextualized embeddings; and
a prediction model to predict, based on the target contextualized embeddings, the prognosis.

3. The computer-implemented method as claimed in claim 2, wherein the prediction model comprises a first neural network to predict, based on the target contextualized embeddings, an event time of the predicted prognosis, and a second neural network to predict, based on the target contextualized embeddings and the predicted event time, the prognosis.

4. The computer-implemented method as claimed in claim 3, wherein the second neural network comprises a plurality of classifiers corresponding to a plurality of possible events.

5. The computer-implemented method as claimed in claim 4, wherein the prediction model comprises an attention network to generate a summary vector based on the target contextualized embeddings and based on the event time predicted by the first neural network and wherein the predicting by the second neural network, based on the target contextualized embeddings and the predicted event time, the prognosis comprises predicting, based on the summary vector, the prognosis.

6. The computer-implemented method as claimed in any of claims 2 to 5, wherein the first encoder model comprises a plurality of self-attention layers.

7. The computer-implemented method as claimed in any of the preceding claims, wherein the second encoder model comprises a plurality of self-attention layers.

8. The computer-implemented method as claimed in any of the preceding claims, wherein the second encoder model has been trained according to a second encoder model training process comprising:
using (S11) the second encoder model to encode a medical history instance to generate contextualized embeddings of the medical history instance;
generating (S12) a vectorial representation of the medical history instance based on the contextualized embeddings of the medical history instance;
using (S13) a decoder model to generate a reconstructed medical history instance based on the vectorial representation;
comparing (S14) the medical history instance with the reconstructed medical history instance; and
adjusting (S15) at least one weight of the second encoder model based on the comparison,
and wherein the ML model has been trained according to an ML model training process comprising:
using (S22) the ML model to predict a prognosis for a subject among the plurality of subjects based on training data comprising medical history information relating to the subject, the medical history information relating to the subject including an indication of a treatment given to the subject;
comparing (S23) the predicted prognosis with a ground truth prognosis in the training data; and
adjusting (S24) at least one weight of the ML model based on the comparison.

9. The computer-implemented method as claimed in claim 8, comprising training the second encoder model according to the second encoder model training process and, after training the second encoder model, training the ML model according to the ML model training process, wherein training the ML model according to the ML model training process comprises initializing (S21) the weights of the first encoder model using the weights of the second encoder model.

10. A computer program which, when run on a computer, causes the computer to carry out a method comprising:
using a machine learning, ML, model to predict a prognosis for a target patient based on medical history information relating to the target patient, the medical history information relating to the target patient including an indication of a treatment to be given to the target patient, wherein the ML model has been trained on training data comprising medical history information relating to a plurality of subjects;
comparing modified medical history information relating to the target patient, comprising the medical history information relating to the target patient and the predicted prognosis, with a plurality of medical history instances, each medical history instance comprising medical history information from among the training data and relating to a subject among the plurality of subjects;
based on the comparison, selecting at least one medical history instance most similar to the modified medical history information relating to the target patient; and
outputting information indicating the predicted prognosis and the selected at least one medical history instance,
wherein comparing the modified medical history information relating to the target patient with the plurality of medical history instances comprises:
using a second encoder model to encode each medical history instance to generate contextualized embeddings of each medical history instance;
generating a vectorial representation of each medical history instance based on the contextualized embeddings of the medical history instance;
using the second encoder model to encode the modified medical history information relating to the target patient to generate contextualized embeddings of the modified medical history information relating to the target patient;
generating a vectorial representation of the modified medical history information relating to the target patient based on the contextualized embeddings of the modified medical history information relating to the target patient; and
comparing the vectorial representation of the modified medical history information relating to the target patient with the vectorial representations of the medical history instances,
wherein the computer program, when run on the computer, causes the computer to:
use a first at least one processor and/or computing device to encode each medical history instance using the second encoder model, generate the vectorial representation of each medical history instance, encode the modified medical history information using the second encoder model, and generate the vectorial representation of the modified medical history information; and
use a second at least one processor and/or computing device to compare the vectorial representation of the modified medical history information relating to the target patient with the vectorial representations of the medical history instances,
wherein the first at least one processor and/or computing device has a larger processing power and/or computing load than the second at least one processor and/or computing device, and wherein the use of the first at least one processor and/or computing device is partially simultaneous with the use of the second at least one processor and/or computing device.

11. A system comprising a plurality of computing devices configured to:
use a machine learning, ML, model to predict a prognosis for a target patient based on medical history information relating to the target patient, the medical history information relating to the target patient including an indication of a treatment to be given to the target patient, wherein the ML model has been trained on training data comprising medical history information relating to a plurality of subjects;
compare modified medical history information relating to the target patient, comprising the medical history information relating to the target patient and the predicted prognosis, with a plurality of medical history instances, each medical history instance comprising medical history information from among the training data and relating to a subject among the plurality of subjects;
based on the comparison, select at least one medical history instance most similar to the modified medical history information relating to the target patient; and
output information indicating the predicted prognosis and the selected at least one medical history instance,
wherein comparing the modified medical history information relating to the target patient with the plurality of medical history instances comprises:
using a second encoder model to encode each medical history instance to generate contextualized embeddings of each medical history instance;
generating a vectorial representation of each medical history instance based on the contextualized embeddings of the medical history instance;
using the second encoder model to encode the modified medical history information relating to the target patient to generate contextualized embeddings of the modified medical history information relating to the target patient;
generating a vectorial representation of the modified medical history information relating to the target patient based on the contextualized embeddings of the modified medical history information relating to the target patient; and
comparing the vectorial representation of the modified medical history information relating to the target patient with the vectorial representations of the medical history instances,
wherein the plurality of computing devices comprises:
a first at least one processor and/or computing device configured to encode each medical history instance using the second encoder model, generate the vectorial representation of each medical history instance, encode the modified medical history information using the second encoder model, and generate the vectorial representation of the modified medical history information; and
a second at least one processor and/or computing device configured to compare the vectorial representation of the modified medical history information relating to the target patient with the vectorial representations of the medical history instances,
wherein the first at least one processor and/or computing device has a larger processing power and/or computing load than the second at least one processor and/or computing device, and wherein the use of the first at least one processor and/or computing device is partially simultaneous with the use of the second at least one processor and/or computing device.
